(19) Europäisches Patentamt
European Patent Office
Office européen des brevets

(11) Numéro de publication: **0 498 707 B1**

(12) **FASCICULE DE BREVET EUROPEEN**

(45) Date de publication de fascicule du brevet: **02.08.95**

(51) Int. Cl.$^6$: **A61K 7/13**

(21) Numéro de dépôt: **92400270.2**

(22) Date de dépôt: **03.02.92**

# (54) Procédé de teinture des fibres kératiniques avec un mono- ou dihydroxyindole et un dérivé carbonylé aromatique non-oxydant et agent de teinture.

(30) Priorité: **04.02.91 FR 9101234**

(43) Date de publication de la demande:
**12.08.92 Bulletin 92/33**

(45) Mention de la délivrance du brevet:
**02.08.95 Bulletin 95/31**

(84) Etats contractants désignés:
**AT BE CH DE DK ES FR GB GR IT LI NL PT SE**

(56) Documents cités:
**EP-A- 0 271 186**
**EP-A- 0 376 776**
**FR-A- 2 626 173**
**GB-A- 2 093 867**
**GB-A- 2 119 411**

(73) Titulaire: **L'OREAL**
**14, rue Royale**
**F-75008 Paris (FR)**

(72) Inventeur: **Grollier, Jean-François**
**16bis Boulevard Morland**
**F-75004 Paris (FR)**

(74) Mandataire: **Casalonga, Axel et al**
**BUREAU D.A. CASALONGA - JOSSE**
**Morassistrasse 8**
**D-80469 München (DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne un nouveau procédé de coloration des fibres kératiniques, et plus particulièrement des fibres kératiniques humaines, telles que les cheveux, mettant en oeuvre au moins un mono- ou dihydroxyindole et au moins un dérivé carbonylé aromatique non-oxydant.

La demanderesse a déjà proposé dans le passé de teindre les fibres kératiniques, et en particulier les cheveux, à l'aide de dérivés hydroxylés de l'indole. Il est en effet connu que la synthèse naturelle des eumélanines à partir de la tyrosine se fait en plusieurs étapes, dont l'une consiste dans la formation du 5,6-dihydroxyindole qui s'oxyde pour former un pigment qui est l'un des constituants principaux de l'eumélanine.

Ainsi, les brevets français de la demanderesse n° 1.166.172 et 1.264.707 en particulier, proposent de colorer les cheveux avec des compositions aqueuses renfermant du 5,6-dihydroxyindole. Il est possible avec de telles compositions de teindre les cheveux dans des nuances claires par une application du produit ou dans des nuances de plus en plus soutenues par superposition des applications.

Ces procédés ne permettent cependant pas' d'obtenir une palette suffisamment large de teintes variées, notamment les teintes dites "à reflets" qui sont particulièrement recherchées en coloration capillaire.

Ont également été décrits des procédés de teinture capillaire mettant en oeuvre des dérivés d'indole, dans lesquels la couleur est révélée par des systèmes oxydants. De tels systèmes oxydants sont notamment décrits dans la demande de brevet européen n° 376.776 et d'autres oxydants connus sont constitués par le peroxyde d'hydrogène associé ou non à un iodure, les periodates, les bromates, les persulfates, les perborates et les nitrites, notamment.

Le document FR-A-2 626 173 décrit un procédé de teinture des fibres kératiniques consistant à appliquer une composition contenant un dérivé d'indole. La couleur est révélée par une système oxydant.

La demanderesse a maintenant mis en évidence que l'on pouvait obtenir, dans un procédé qui ne nécessite pas d'autre révélation oxydante que celle de l'oxygène de l'air, des teintes variées très naturelles ou très en reflets, sans présenter les inconvénients de la technique antérieure. En effet, en teinture capillaire, il est généralement difficile d'obtenir des teintes naturelles et une uniformité de couleur sur les cheveux longs et/ou sensibilisés par une permanente, coloration ou décoloration, et sur toute leur longueur.

Le procédé selon l'invention est un procédé de coloration des fibres kératiniques, en particulier des cheveux, mettant en oeuvre, dans un processus à deux temps, l'application d'au moins un dérivé d'indole, cette application étant précédée ou suivie par celle d'au moins un dérivé carbonylé aromatique non-oxydant.

Ce procédé permet d'obtenir une bonne couverture des cheveux blancs dans une nuance naturelle uniforme sur l'ensemble de la chevelure, sans altérer les propriétés mécaniques des cheveux. Ces teintures sont peu sélectives et présentent en outre une bonne résistance à la transpiration, aux lavages, à la lumière, aux agressions chimiques et atmosphériques.

Le procédé de teinture des fibres kératiniques est caractérisé en ce que l'on applique sur les fibres :

a) une composition (A) contenant, dans un milieu approprié pour la teinture, au moins un mono- ou dihydroxyindole, cette application étant précédée ou suivie par l'application de

b) une composition (B) contenant, dans un milieu approprié pour la teinture, au moins un dérivé carbonylé aromatique choisi parmi les hydroxyacétophénones, les hydroxybenzophénones, les 2-hydroxy 1,4-benzoquinones, les hydroxy 1,4-naphtoquinones, les amino 1,4-naphtoquinones, les hydroxy 9,10-anthraquinones et les amino 9,10-anthraquinones.

Un autre objet de l'invention est constitué par un agent de teinture à plusieurs composants, de préférence sous forme de "kit de teinture" ou "nécessaire de teinture" pour la mise en oeuvre du procédé selon l'invention.

On utilise comme mono- ou dihydroxyindole, selon l'invention, les composés de formule (I) :

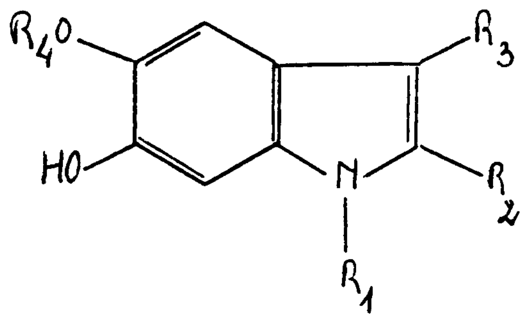

(I)

dans laquelle:

$R_1$, $R_3$ et $R_4$, identiques ou différents, représentent un atome d'hydrogène ou un groupement alkyle en $C_1$-$C_4$;

$R_2$ désigne un atome d'hydrogène, un groupement alkyle en $C_1$-$C_4$ ou un groupement carboxyle; et

les sels correspondants de métaux alcalins, alcalino-terreux, d'ammonium et d'amines de ces composés.

Le potentiel d'oxydo-réduction des dérivés indoliques de formule (I), mesuré à pH 7 sur électrode de carbone vitreux par voltamétrie est, selon l'invention, inférieur à 200 mV.

Parmi ces indoles, les composés préférés selon l'invention sont choisis parmi :

le 5,6-dihydroxyindole,
le 2-méthyl 5,6-dihydroxyindole,
le 3-méthyl 5,6-dihydroxyindole,
le 1-méthyl 5,6-dihydroxyindole,
le 2,3-diméthyl 5,6-dihydroxyindole,
le 5-méthoxy 6-hydroxyindole, et
le 2-carboxy 5,6-dihydroxyindole.

Les dérivés carbonylés aromatiques non-oxydants sont notamment choisis parmi les composés suivants :

- les hydroxyacétophénones ou hydroxybenzophénones qui sont choisies parmi les composés de formule (II) :

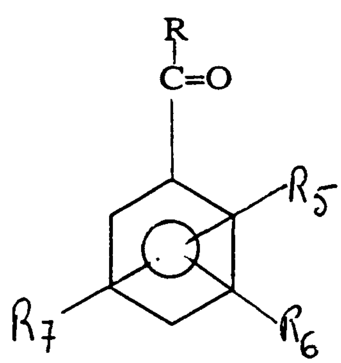

(II)

dans laquelle:

$R_5$, $R_6$, $R_7$, identiques ou différents, désignent un atome d'hydrogène, un groupement hydroxy ou méthoxy, l'un au moins parmi $R_5$, $R_6$ et $R_7$,, désignant hydroxy,

R représente un groupement méthyle ou $R_8$, $R_8$ représentant un radical

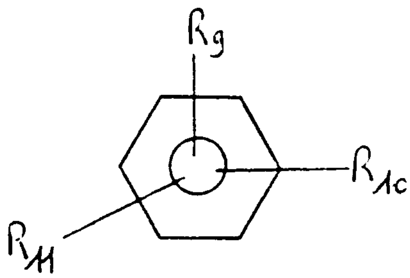

où $R_9$, $R_{10}$ et $R_{11}$, identiques ou différents, représentent un atome d'hydrogène, un groupement hydroxy ou méthoxy;

- les 2-hydroxy 1,4-benzoquinones qui sont choisies parmi les composés de formule (III) :

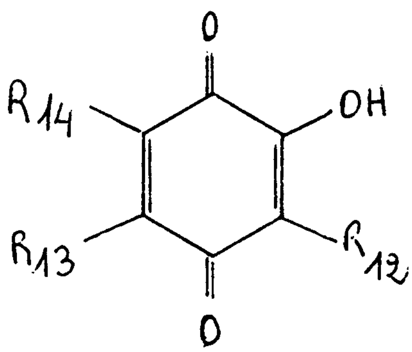

(III)

dans laquelle:

$R_{12}$, $R_{13}$ et $R_{14}$, identiques ou différents, représentent un atome d'hydrogène, un groupement alcoxy en $C_1$-$C_4$, hydroxy, alkyle en $C_1$-$C_4$, amino, aminoalkyle en $C_1$-$C_4$ ou parahydroxyphényle;

- les hydroxy 1,4-naphtoquinones ou amino 1,4-naphtoquinones qui sont choisies parmi les composés de formule (IV) :

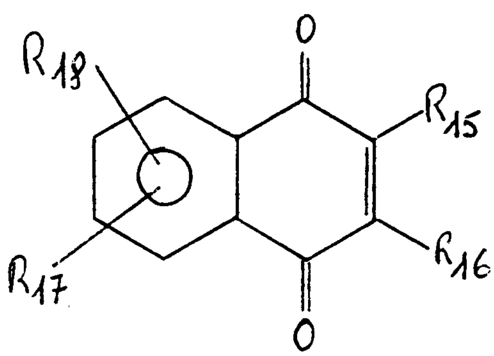

(IV)

dans laquelle:

$R_{15}$ et $R_{16}$, identiques ou différents, représentent un atome d'hydrogène ou de chlore, un groupement hydroxy, méthoxy, méthyle, isopentényle, acétyle, $-SCH_2COOH$, ou un groupement $-NR_{19}R_{20}$, où $R_{19}$ et $R_{20}$, identiques ou différents, représentent un atome d'hydrogène ou un groupement méthyle ou hydroxyéthyle,

$R_{17}$ et $R_{18}$, identiques ou différents, représentent un atome d'hydrogène, un groupement hydroxy, méthoxy ou méthyle,

l'un au moins parmi $R_{15}$, $R_{16}$, $R_{17}$ et $R_{18}$ représentant hydroxy ou l'un au moins parmi $R_{15}$ et $R_{16}$ représentant $-NR_{19}R_{20}$;

- les hydroxy 9,10-anthraquinones ou amino 9,10-anthraquinones qui sont choisies parmi les composés de formule (V) :

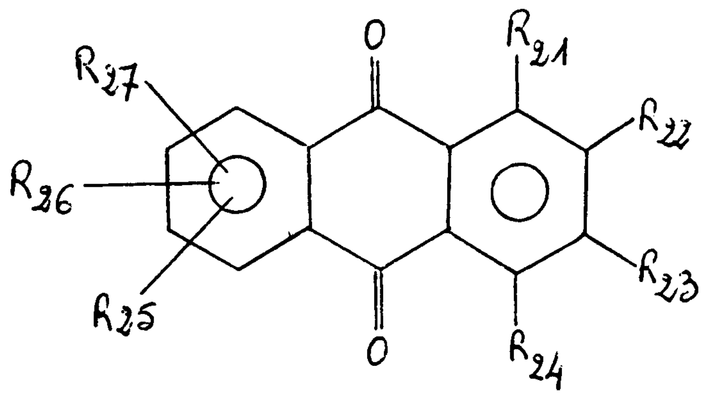

(V)

dans laquelle :

$R_{21}$ à $R_{27}$, identiques ou différents, représentent un atome d'hydrogène ou de chlore, un groupement hydroxy, méthyle, $SO_3M$, COOM ou $NR_{28}R_{29}$,

où M désigne un atome d'hydrogène, Na ou K , et $R_{28}$ et $R_{29}$, identiques ou différents, représentent un atome d'hydrogène, un groupement alkyle en $C_1$-$C_4$ ou monohydroxyalkyle en $C_1$-$C_4$,

l'un au moins parmi $R_{21}$ à $R_{27}$ représentant un groupement hydroxy ou $NR_{28}R_{29}$.

Parmi ces dérivés carbonylés non-oxydants, selon l'invention, on peut citer les composés suivants :
la 2-hydroxyacétophénone,
la 2,5-dihydroxyacétophénone,
la 2,6-dihydroxyacétophénone,
la 4-hydroxy 3-méthoxyacétophénone,
la 3,4,5-trihydroxyacétophénone,
la 2',2'-dihydroxybenzophénone,
la 2-hydroxybenzophénone,
la 2,2',4,4'-tétrahydroxybenzophénone, et
la 3,4,2',4',6'-pentahydroxybenzophénone;
la 2,5-dihydroxy 1,4-benzoquinone,
la 2,5-dihydroxy 3-éthyl 1,4-benzoquinone,
la 2,5-dihydroxy 3,6-diméthoxy 1,4-benzoquinone,
la 2,5-dihydroxy 3-méthyl 6-isopropyl 1,4-benzoquinone,
la 2,5-dihydroxy 3,6-bis-p-hydroxyphényl 1,4-benzoquinone,
la 2-hydroxy 3-méthyl 6-méthoxy 1,4-benzoquinone,
la 2-hydroxy 3-méthyl 5,6-diméthoxy 1,4-benzoquinone,
la 2-hydroxy 3-méthoxy 6-méthyl 1,4-benzoquinone, et
la 2,3,5,6-tétrahydroxy 1,4-benzoquinone;
la 2-hydroxy 3-isopentényl 1,4-naphtoquinone,
la 2-hydroxy 3-amino 7-méthoxy 1,4-naphtoquinone,
la 2-hydroxy 3-N-méthylamino 1,4-naphtoquinone,
la 2-hydroxy 6-méthoxy 1,4-naphtoquinone,
la 2-hydroxy 5-méthoxy 1,4-naphtoquinone,
la 2-hydroxy 8-méthoxy 1,4-naphtoquinone,
la 2-hydroxy 5,8-diméthoxy 1,4-naphtoquinone,
la 2-hydroxy 3-acétyl 1,4-naphtoquinone,
la 5-hydroxy 2-méthoxy 1,4-naphtoquinone,
la 5-hydroxy 3-diméthylamino 1,4-naphtoquinone,
la 5-hydroxy 3-méthoxy 1,4-naphtoquinone,
la 5-hydroxy 2-méthyl 1,4-naphtoquinone,
la 5-hydroxy 2-méthyl 3-chloro 1,4-naphtoquinone,
la 5-hydroxy 2-carboxyméthylthio 1,4-naphtoquinone,
la 5-hydroxy 3-carboxyméthylthio 1,4-naphtoquinone,
la 5,8-dihydroxy 1,4-naphtoquinone,
la 5,8-dihydroxy 2-chloro 1,4-naphtoquinone,
la 2,7-dihydroxy 1,4-naphtoquinone,
la 5,7-dihydroxy 1,4-naphtoquinone,

la 3,5-dihydroxy 2-méthyl 1,4-naphtoquinone,
la 3,5-dihydroxy 1,4-naphtoquinone,
la 2,6-dihydroxy 1,4-naphtoquinone,
la 2,3-dihydroxy 5-méthoxy 1,4-naphtoquinone,
la 2,3-dihydroxy 7-méthoxy 1,4-naphtoquinone,
la 3,6,8-trihydroxy 1,4-naphtoquinone,
la 2,3,5-trihydroxy 1,4-naphtoquinone,
la 2,6,7-trihydroxy 1,4-naphtoquinone,
la 2,3,5,8-tétrahydroxy 1,4-naphtoquinone, et
la 1,4-dihydroxy 9,10-anthraquinone,
la 1,5-dihydroxy 9,10-anthraquinone,
la 1,8-dihydroxy 9,10-anthraquinone,
la 2,6-dihydroxy 9,10-anthraquinone,
la 1,2,5,8-tétrahydroxy 9,10-anthraquinone,
la 1,2,3,5,6,7-hexahydroxy 9,10-anthraquinone,
la 1,8-dihydroxy 3-méthyl 9,10-anthraquinone,
la 1,3,8-trihydroxy 6-méthyl 9,10-anthraquinone,
la 5,8-dichloro 1,4-dihydroxy 9,10-anthraquinone,
le sel de sodium de l'acide 1,2-dihydroxy 9,10-anthraquinone 3-sulfonique,
le sel de sodium de l'acide 1,2,4-trihydroxy 9,10-anthraquinone 3-sulfonique,
la 1-hydroxyanthraquinone,
la 2-hydroxyanthraquinone,
la 1,8-dihydroxy 3-carboxy 9,10-anthraquinone,
la 1,4,5,8-tétraaminoanthraquinone, et
la 1,4-diaminoanthraquinone.

Parmi ces composés, on préfère particulièrement :

- la 5,8-dihydroxy 1,4-naphtoquinone,
- la 1,2,5,8-tétrahydroxyanthraquinone, et
- la 1,4,5,8-tétraaminoanthraquinone.

Parmi les composés carbonylés non-oxydants, on peut citer aussi les composés suivants :

la 2,5-dihydroxy 3-méthyl 1,4-benzoquinone,
la 2,5-dihydroxy 3-méthoxy 6-méthyl 1,4-benzoquinone,
la 2-hydroxy 1,4-naphtoquinone,
la 2-hydroxy 3-méthyl 1,4-naphtoquinone,
la 2,5-dihydroxy 1,4-naphtoquinone,
la 2,3-dihydroxy 1,4-naphtoquinone,
la 2,5,7-trihydroxy 1,4-naphtoquinone,
la 5-hydroxy 1,4-naphtoquinone,
la 2,5,8-trihydroxy 1,4-naphtoquinone,
la 2-hydroxy 3-méthoxy 1,4-naphtoquinone,
la 2-carboxy 1-méthyl 3,5,6,8-tétrahydroxy anthraquinone,
la 1,2-dihydroxyanthraquinone,
la 1,2,4-trihydroxyanthraquinone, et
la 3-carboxy 1,2,4-trihydroxyanthraquinone.

Les composés selon l'invention sont connus et peuvent être obtenus par synthèse ou, le cas échéant, à partir des organismes les élaborant ou des végétaux les contenant quand ils ont une origine naturelle. Dans ce dernier cas, les produits contenant ces colorants peuvent être utilisés, soit sous forme d'extraits, soit sous forme d'homogénéisats de tout ou partie des organismes ou des végétaux.

Les compositions (A) et (B) et notamment la composition (B) utilisable dans le procédé de l'invention, peuvent également contenir d'autres colorants naturels directs habituellement utilisés pour la teinture des fibres kératiniques. Ainsi, on peut utiliser également l'isatine, la purpurogalline, la pyocyanine, la curcumine, l'indigo, l'hématoxyline, la brasiline, la riboflavine, la bixine, le $\beta$-carotène, la rutine, la quercétine. On peut également utiliser des colorants nitrés sur le cycle benzénique.

Selon l'invention, la proportion en mono- ou dihydroxyindole de formule (I) présente dans la composition (A), est comprise entre 0,05 et 5% en poids de cette composition et de préférence comprise entre 0,05 et 3% en poids par rapport au poids total de cette composition (A).

La proportion en dérivé carbonylé aromatique non-oxydant contenu dans la composition (B) est, selon l'invention, comprise entre 0,01 et 5% en poids et de préférence entre 0,05 et 3% en poids par rapport au

poids total de la composition (B).

Selon l'invention, les compositions (A) et (B) aqueuses ou anhydres, sont utilisées sous forme de liquides plus ou moins épaissis, ou encore sous forme de crèmes, de gels aqueux ou anhydres, d'huiles ou de poudres à diluer avec un liquide au moment de l'emploi encore appelé "cataplasme".

Dans une première forme de réalisation de l'invention, le milieu approprié pour la teinture utilisé pour les deux compositions (A) et (B), est aqueux. Son pH peut varier entre 2 et 10, et il est de préférence compris entre 3 et 9,5, cette valeur pouvant être ajustée à la valeur désirée à l'aide d'agents alcalinisants ou d'agents acidifiants connus.

Ces compositions peuvent contenir des agents tensio-actifs anioniques, cationiques, non-ioniques, amphotères ou des mélanges de tels tensio-actifs. A titre d'agents tensio-actifs, on peut citer plus particulièrement les savons, les alkylbenzènesulfonates, les alkylnaphtalènesulfonates, les sulfates ou éthers sulfates ou sulfonates d'alcools gras, les sels d'ammonium quaternaires, les diéthanolamides d'acides gras, les acides ou alcools ou amides polyoxyéthylénés ou polyglycérolés, les alkylphénols polyoxyéthylénés ou polyglycérolés, les alkylpolyglycosides.

Selon l'invention, les tensio-actifs sont présents dans les compositions utilisables pour la mise en oeuvre du procédé, dans des proportions comprises entre 0,1 et 55% en poids des compositions, et de préférence entre 1 et 40% en poids par rapport au poids total de chacune de ces compositions.

Le milieu approprié pour la teinture, aqueux, peut en outre renfermer des solvants organiques tels que par exemple les alcanols inférieurs tels que l'éthanol ou l'isopropanol, les polyols tels que le glycérol, les glycols ou éthers de glycols, comme l'éthylèneglycol, le propylèneglycol, l'éther monobutylique de l'éthylèneglycol, le monoéthyléther et le monométhyléther du diéthylèneglycol, ainsi que des produits analogues ou leurs mélanges.

De préférence, ces solvants sont alors présents dans des proportions allant de 1 à 60% en poids par rapport au poids total de la composition, et plus particulièrement comprises entre 3 et 30% en poids.

Les compositions pour la mise en oeuvre du procédé selon l'invention, peuvent également contenir des polymères anioniques, non-ioniques, cationiques ou amphotères ou leurs mélanges, dans des proportions comprises entre 0,1 et 5% en poids par rapport au poids total de la composition.

Les compositions pour la mise en oeuvre du procédé selon l'invention peuvent encore comporter des agents épaississants, des parfums, des agents séquestrants, des agents filmogènes, des agents de traitement des fibres kératiniques, des agents dispersants, des agents de conditionnement, des agents conservateurs, des agents opacifiants, des agents de gonflement des fibres kératiniques, des agents de pénétration, des agents antioxydants et des tampons.

Dans cette première forme de réalisation de l'invention, les compositions peuvent être épaissies avec des agents épaississants. Parmi les agents épaississants possibles, on peut citer par exemple l'alginate de sodium, la gomme arabique, la gomme de guar ou de caroube, la gomme de xanthane, les pectines, les dérivés de la cellulose tels que la méthylcellulose, l'hydroxyéthylcellulose, l'hydroxyméthylcellulose, l'hydroxypropylcellulose, la carboxyméthylcellulose et les polymères divers ayant une fonction épaississante tels que les dérivés d'acide acrylique. On peut également utiliser des agents épaississants minéraux tels que la bentonite.

Lorsque les compositions comprennent des agents épaississants, ceux-ci sont présents dans des proportions comprises entre 0,1 et 5% en poids par rapport au poids total de la composition et en particulier dans des proportions comprises entre 0,5 et 3% en poids.

En outre, ces compositions peuvent contenir d'autres adjuvants habituellement utilisés dans les compositions pour la teinture des fibres kératiniques, en particulier la teinture des cheveux, tels que des agents de pénétration, des agents séquestrants, des agents antioxydants, des tampons, des parfums, etc.

Une forme préférée de l'invention consiste à utiliser, pour la composition (B), un milieu anhydre tel qu'il est décrit dans la demande de brevet français n° 2.526.031. Par milieu anhydre, on entend un milieu quine contient pas plus de 1% d'eau.

Selon cette variante, le milieu anhydre est constitué par un mélange d'au moins un solvant anhydre et d'au moins un ou plusieurs agents tensio-actifs anhydres, la composition comportant au moins 15% de solvant et au moins 20% d'agent tensio-actif.

Selon l'invention, on peut utiliser les solvants cosmétiquement acceptables choisis parmi les monoalcools saturés en $C_2$-$C_{20}$ tels que l'éthanol, l'isopropanol, l'alcool cétylique ou l'octyldodécanol; les polyols tels que les alcoylèneglycols comme l'éthylèneglycol, le propylèneglycol, le glycérol, le diéthylèneglycol; les éthers de glycols tels que les mono-, di- et triéthylèneglycolmonoalcoyléthers comme par exemple l'éthylèneglycolmonoéthyléther, l'éthylèneglycolmonobutyléther, le diéthylèneglycolmonoéthyléther; des esters comme par exemple l'acétate de monométhyléther de l'éthylèneglycol, l'acétate de monoéthyléther de l'éthylèneglycol; les esters d'acides gras et d'alcools inférieurs saturés comme le myristate ou le palmitate

d'isopropyle, et leurs mélanges.

Parmi ces solvants, les solvants particulièrement préférés sont choisis parmi l'éthanol, l'alcool cétylique, le propylèneglycol, l'éthylèneglycolmonoéthyléther ou l'éthylèneglycolmonobutyléther.

Selon cette variante, les agents tensio-actifs sont choisis parmi les agents tensio-actifs anhydres de type anionique, non-ionique, cationique, amphotère ou leurs mélanges. On peut citer plus particulièrement les alcools gras polyoxyéthylénés, les alkylphénols ou naphtols polyoxyéthylénés, les halogénures de monoalkyltriméthylammonium, les halogénures de dialkyldiméthylammonium, les savons, les alcools gras polyglycérolés. Les agents tensio-actifs particulièrement préférés sont les agents tensio-actifs non-ioniques.

En outre, ces compositions anhydres peuvent contenir un agent alcalin ou acidifiant, anhydre tel que par exemple l'acide citrique, l'acide ascorbique, l'acide acétique, l'acide lactique et des alcanolamines telles que, de préférence, celles qui sont totalement substituées sur le groupement amine comme le diméthylaminoéthanol.

Les compositions anhydres peuvent en outre contenir d'autres additifs anhydres tels qu'ils sont utilisés en cosmétique. On peut citer parmi ces additifs, les parfums, les agents épaississants, les agents de traitement des cheveux, les agents anti-oxydants, les huiles végétales ou minérales, les agents conservateurs et les sels organiques.

Ces compositions selon l'invention peuvent être appliquées telles quelles sur les cheveux mouillés ou, selon une seconde forme d'application, elles peuvent être diluées tout juste avant l'emploi. Dans ce dernier cas, au moment de la teinture, les compositions sont diluées avec une solution aqueuse, de telle sorte que le rapport en poids entre la composition anhydre conforme à l'invention et la solution aqueuse soit compris entre 0,25 et 2.

La solution aqueuse utilisée pour la dilution peut être constituée par de l'eau pure, mais également par tout autre liquide aqueux complexe, plus ou moins épaissi, comme un support habituellement utilisé dans les compositions tinctoriales pour cheveux.

Dans ce cas, les composants du milieu cosmétique peuvent être tous types d'ingrédients cosmétiquement acceptables, anhydres ou pas, tels qu'ils sont habituellement utilisés dans ce type de composition et décrits de façon générale ci-dessus.

Dans une autre variante particulièrement préférée de réalisation de la composition (B), conformément à l'invention, celle-ci est appliquée sous forme de cataplasme, c'est-à-dire sous forme de poudres à diluer avec un liquide au moment de l'emploi.

Dans cette forme de réalisation, les colorants sont préparés sous forme de poudre stable au stockage et introduits dans un milieu solide pouvant être constitué de poudres, de farines, de substances amylacées ou mucilagineuses. Au moment de l'emploi, on dilue la poudre avec un liquide adéquat de façon à former un mélange ayant une consistance appropriée à l'application sur la tête.

Les poudres utilisées dans les cataplasmes conformes à cette variante de l'invention, peuvent être constituées de substances insolubles telles que des silices, des végétaux, des argiles, des végétaux pulvérisés après extraction de leur principe actif par un solvant ou encore des végétaux ou animaux contenant les dérivés carbonylés aromatiques non-oxydants tels que définis ci-dessus.

Le liquide de dilution peut être constitué par de l'eau ou des mélanges eau/solvant(s) cosmétiquement acceptables tels que des alcools ou des glycols, ou encore des huiles.

Le milieu liquide est ajouté à la poudre dans des proportions telles qu'après mélange, on obtienne une pâte ayant une viscosité comprise entre 0,3 et 5 Pa.s.

L'agent de teinture pour fibres kératiniques utilisé pour la mise en oeuvre du procédé conforme à l'invention, est essentiellement caractérisé par le fait qu'il comprend au moins deux composants séparés (A) et (B) ayant la composition indiquée pour les compositions (A) et (B) définies ci-dessus.

On peut conditionner les différentes compositions (A) et (B) dans un dispositif à plusieurs compartiments encore appelé "kit de teinture" ou "nécessaire de teinture" ou "agent de teinture" comportant tous les composants destinés à être appliqués pour une même teinture sur les fibres kératiniques, en particulier les cheveux, en applications successives avec ou sans prémélange.

De tels dispositifs selon l'invention peuvent comporter un premier compartiment contenant la composition (A) comportant le mono- ou dihydroxyindole de formule (I) et un second compartiment contenant la composition (B) comportant le ou les dérivés carbonylés aromatiques non-oxydants de formules (II), (III), (IV) ou (V).

Un autre variante peut consister à stocker la composition (A) et/ou la composition (B) en milieu solvant anhydre et à prévoir alors un troisième compartiment contenant un milieu aqueux approprié pour la teinture et cosmétiquement acceptable; on mélange dans ce cas juste avant l'emploi le contenu du troisième compartiment avec le contenu de l'un et/ou l'autre des deux autres compartiments contenant les compositions anhydres (A) et/ou (B).

Le procédé selon l'invention peut être mis en oeuvre par mélange, juste avant l'emploi, des compositions (A) et (B), le mélange résultant étant appliqué sur les cheveux pendant 2 à 60 minutes; cette application est suivie d'un rinçage et éventuellement d'un lavage de la chevelure.

Le procédé selon l'invention est de préférence mis en oeuvre en appliquant, dans un premier temps, la composition (A) et, dans un second temps, la composition (B) en effectuant éventuellement un rinçage entre les deux étapes.

Il peut être utilisé notamment pour teindre les cheveux humains, partiellement ou totalement gris ou blancs, pemanentés ou non, ou défrisés, les cheveux fortement ou légèrement décolorés, et éventuellement permanentés.

On applique la composition (A) à une température compatible avec l'application sur la tête, c'est-à-dire comprise entre 25 et 40 °C, pendant 2 à 60 minutes et de préférence 5 à 30 minutes et, après ou sans rinçage intermédiaire, on fait suivre l'application de la composition (A) par l'application de la composition (B) qui contient le dérivé carbonylé aromatique non-oxydant. La composition (B) est maintenue au contact des cheveux pendant 5 à 60 minutes, et de préférence 10 à 40 minutes, la température de teinture étant également comprise entre 25 et 40 °C.

Les exemples suivants sont destinés à illustrer l'invention sans pour autant présenter un caractère limitatif.

## EXEMPLES 1 à 8

| Composition (A) en g MA | 1 | 2 | 3 | 4 |
|---|---|---|---|---|
| 5,6-dihydroxyindole | 0,2 | 0,5 | 0,5 | 0,5 |
| Alcool éthylique | 10 | 10 | 10 | 10 |
| Alkylpolyglycoside vendu sous la dénomination ORAMIX CG 110 à 60% de MA par la Société AQUALON | 2,1 | 2,1 | 2,1 | 2,1 |
| Hydroxyéthylcellulose vendue sous la dénomination NATROSOL 250 HHR par la Société AQUALON | 1 | 1 | 1 | 1 |
| Acide tartrique | 0,3 | 0,3 | 0,3 | 0,3 |
| Conservateur | qs | qs | qs | qs |
| Triéthanolamine | 3,75 | 3,75 | 3,75 | 3,75 |
| Eau qsp | 100 | 100 | 100 | 100 |
| Temps de pose (en mn) | 15 | 10 | 5 | 20 |
| Rinçage intermédiaire | oui | oui | non | oui |

EXEMPLES 1 à 8 (suite)

| | 5 | 6 | 7 | 8 |
|---|---|---|---|---|
| Composition (A) en g MA | | | | |
| 5,6-dihydroxyindole | 0,5 | 0,5 | 0,5 | |
| 2-carboxy 5,6-dihydroxyindole | | | 0,05 | |
| 2-méthyl 5,6-dihydroxyindole, HBr | | | | 1 |
| Alcool éthylique | 10 | 10 | 10 | 10 |
| Alkylpolyglycoside vendu sous la dénomination ORAMIX CG 110 à 60% de MA par la Société AQUALON | 2,1 | 2,1 | 2,1 | 2,1 |
| Hydroxyéthylcellulose vendue sous la dénomination NATROSOL 250 HHR par la Société AQUALON | 1 | 1 | 1 | 1 |
| Acide tartrique | 0,3 | 0,3 | 0,3 | 0,3 |
| Conservateur | qs | qs | qs | qs |
| Triéthanolamine | 3,75 | 3,75 | 3,75 | 3,75 |
| Eau qsp | 100 | 100 | 100 | 100 |
| Temps de pose (en mn) | 20 | 10 | 10 | 15 |
| Rinçage intermédiaire | oui | non | oui | oui |

On applique 10 g de la composition (A) sur 3 g de cheveux pendant le temps indiqué ci-dessus, suivi ou non d'un rinçage.

On applique ensuite 8 g de la composition (B) des exemples 1 à 5 ou 5 g de la composition (B) des exemples 6 à 8 pendant le temps indiqué dans les tableaux ci-après, les compositions (B) des exemples 1 à 5 étant diluées avant l'emploi avec 1,5 fois leurs poids en eau, celles des exemples 6 à 8 étant diluées avec 3 fois leurs poids en eau.

Les cheveux sont ensuite rincés, lavés puis séchés.

On obtient en final une coloration uniforme sur toute la chevelure dont la nuance est indiquée dans les tableaux ci-après.

TABLEAU

| | 1 | 2 | 3 | 4 |
|---|---|---|---|---|
| Composition (B) en g MA | | | | |
| 2-hydroxy 1,4-naphtoquinone | 1 | 1 | | |
| 2-hydroxy 3-méthoxy 1,4-naphtoquinone | | | | 2,5 |
| 2-hydroxy 3-méthyl 1,4-naphtoquinone | 0,15 | 0,15 | 0,5 | |
| 2,5-dihydroxy 3-méthoxy 6-méthyl 1,4-benzoquinone | | | 0,3 | |
| Isatine | | | 1,3 | 0,3 |
| Alcool cétylique | 0,3 | 0,3 | 0,3 | 0,3 |
| Acide citrique | 1 | 1 | 1 | 1 |
| Chlorure de diméthyl distéaryl ammonium | 1 | 1 | 1 | 1 |
| Hydroxypropylcellulose | 2,2 | 2,2 | 2,2 | 1,5 |
| Alcool éthylique | 28,5 | 28,5 | 28,5 | 28,5 |
| ß-naphtol oxyéthyléné à 7 moles d'oxyde d'éthylène | 15 | 15 | 15 | 15 |
| Nonylphénol oxyéthyléné à 9 moles d'oxyde d'éthylène qsp | 100 | 100 | 100 | 100 |
| Temps de pose (en mn) | 10 | 10 | 15 | 10 |

TABLEAU (suite)

| | 1 | 2 | 3 | 4 |
|---|---|---|---|---|
| **Nuances obtenues**<br><br>sur cheveux gris à 90% blanc (ex. 1-3)<br>sur cheveux châtain à 30% blanc (ex. 4) | blond cuivré doré | blond beige cuivré doré | blond beige irisé légèr. cendré | châtain un peu plus soutenu à reflets auburn |

TABLEAU (suite)

| | 5 | 6 | 7 | 8 |
|---|---|---|---|---|
| Composition (B) en g MA | | | | |
| 2-hydroxy 1,4-naphtoquinone | | 0,5 | | |
| 1,2,4-trihydroxy 9,10-anthraquinone | | | 4 | |
| 2-hydroxy 3-méthyl 1,4-naphtoquinone | 0,4 | | | |
| 2',5'-dihydroxyacétophénone | | | | 2 |
| Isatine | 2 | | | |
| Gomme de caroube vendue sous la dénomination VIDOGUM L 175 par la Société SANOFI Bio Industrie | | 3 | 3 | 3 |
| Acide citrique | 1 | 4 | 4 | 4 |
| Poudre de résidus d'épuisement de saponaire de granulométrie inférieure à 90 micromètres | | 35 | 35 | 35 |
| Lait écrémé en poudre qsp | | 100 | 100 | 100 |
| Alcool cétylique | 0,3 | | | |

TABLEAU (suite et fin)

| | 5 | 6 | 7 | 8 |
|---|---|---|---|---|
| Chlorure de diméthyl distéaryl ammonium | 1 | | | |
| Hydroxypropylcellulose | 1,5 | | | |
| Alcool éthylique | 28,5 | | | |
| ß-naphtol oxyéthyléné à 7 moles d'oxyde d'éthylène | 15 | | | |
| Nonylphénol oxyéthyléné à 9 moles d'oxyde d'éthylène qsp | 100 | | | |
| Temps de pose (en mn) | 10 | 30 | 30 | 30 |
| **Nuances obtenues**<br>sur cheveux gris à 90% blanc (ex. 6-8)<br>sur cheveux blonds à 30% blanc (ex. 5) | blond un peu plus soutenu à reflets dorés | blond cendré irisé | blond cendré irisé | blond naturel cendré mat |

EXEMPLE 9

COMPOSITION (A) :

- 5,6-dihydroxyindole 0,5 g
- Alcool éthylique 10,0 g
- Hydroxypropylcellulose 1,0 g
- Alkyléther de glucoside de formule :

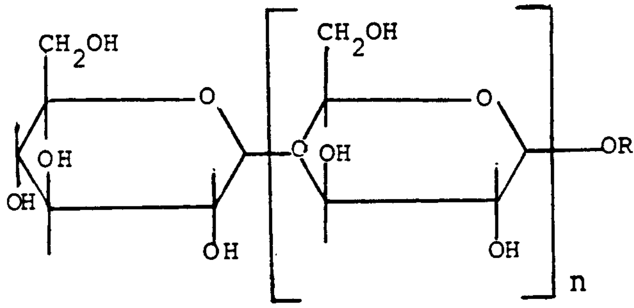

(n=1 à 5, R désigne alkyle en $C_8$-$C_{10}$) vendu à 60% de matière active (MA) sous la dénomination TRITON CG 110 par la Société ROHM & HAAS 2,1 g MA
- Triéthanolamine 3,75 g
- Acide tartrique 0,3 g
- pH=8,5
- Conservateur qs
- Eau qsp 100,0 g

COMPOSITION (B) :

| | |
|---|---|
| - 2-hydroxy 1,4-naphtoquinone | 0,5 g |
| - Poudre de résidus d'épuisement de saponaire de granulométrie inférieure à 90 micromètres | 35,0 g |
| - Gomme de caroube vendue sous la dénomination VIDOGUM L 175 par la Société SANOFI BIO INDUSTRIE | 3,0 g |
| - Acide citrique | 4,0 g |
| - Lait écrémé en poudre qsp | 100,0 g |

La composition (B) est diluée par trois fois son poids en eau, puis appliquée sur des cheveux à 90% de blancs, à raison de 15 g sur 3 g de cheveux. Après un temps de pose de 30 minutes, on rince puis on applique 15 g de la composition (A). On la laisse poser 10 minutes.

Après rinçage, lavage, puis rinçage, les cheveux sont colorés en blond clair cendré doré.

EXEMPLES 10 à 13

On applique 10 g de la composition (A) sur 3 g de cheveux pendant le temps indiqué dans le tableau ci-après, suivi d'un rinçage.

TABLEAU

| | 10 | 11 | 12 | 13 |
|---|---|---|---|---|
| Composition (A) en g MA | | | | |
| 1-méthyl 5,6-dihydroxyindole | 1,5 | | | |
| 2,3-diméthyl 5,6-dihydroxyindole,HBr | | 0,8 | | |
| 5-méthoxy 6-hydroxyindole | | | | 1 |
| 3-méthyl 5,6-dihydroxyindole | | | 0,2 | |
| Alcool éthylique | 10 | 10 | 10 | 10 |
| Alkylpolyglycoside vendu sous la dénomination ORAMIX CG 110 à 60% de MA par la Société AQUALON | 2,1 | 2,1 | 2,1 | 2,1 |
| Hydroxyéthylcellulose vendue sous la dénomination NATROSOL 250 HHR par la Société AQUALON | 1 | 1 | 1 | 1 |
| Acide tartrique | 0,3 | 0,3 | 0,3 | 0,3 |
| Conservateur | qs | qs | qs | qs |
| Triéthanolamine | 3,75 | 3,75 | 3,75 | 3,75 |
| Eau qsp | 100 | 100 | 100 | 100 |
| Temps de pose (en mn) | 15 | 15 | 15 | 15 |
| Rinçage intermédiaire | oui | oui | oui | oui |

On applique ensuite 8 g de la composition (B) de l'exemple 10 ou 5 g de la composition (B) des exemples 11 à 13 pendant le temps indiqué dans le tableau ci-après, la composition (B) de l'exemple 10 étant diluée avant l'emploi avec 1,5 fois son poids en eau, celles des exemples 11 à 13 étant diluées avec 3 fois leurs poids en eau.

Les cheveux sont ensuite rincés puis séchés.

On obtient en final une coloration uniforme sur toute la chevelure dont la nuance est indiquée dans le tableau ci-après.

TABLEAU

| | 10 | 11 | 12 | 13 |
|---|---|---|---|---|
| Composition (B) en g MA | | | | |
| 5,8-dihydroxy 1,4-naphtoquinone | 0,5 | | | |
| 2,2'-dihydroxybenzophénone | | | 0,5 | |
| 1,2,5,8-tétrahydroxyanthraquinone | | 3,5 | | 3,5 |
| Gomme de caroube vendue sous la dénomination VIDOGUM L 175 par la Société SANOFI BIO INDUSTRIE | | | 3 | |
| Carbonate de sodium | | 3 | | 3 |
| Poudre de résidus d'épuisement de saponaire de granulométrie inférieure à 90 micromètres | | 35 | 35 | 35 |
| Lait écrémé en poudre qsp | | 100 | 100 | 100 |
| Alcool cétylique | 0,3 | | | |
| Acide citrique | 1 | | | |
| Chlorure de diméthyl distéaryl ammonium | 1 | | | |
| Hydroxypropylcellulose | 2,2 | | | |
| Alcool éthylique | 28,5 | | | |
| β-naphtol oxyéthyléné à 7 moles d'oxyde d'éthylène | 15 | | | |
| Parfum, conservateurs | qs | qs | qs | qs |

| | | | | |
|---|---|---|---|---|
| Nonylphénol oxyéthyléné à 9 moles d'oxyde d'éthylène qsp | 100 | | | |
| Temps de pose (en mn) | 30 | 30 | 30 | 30 |
| **Nuances obtenues** | | | | |
| sur cheveux naturels gris à 90% blanc | blond clair beige irisé | blond foncé cendré mat | cendré mat moyen | |
| sur cheveux naturels gris à 90% blanc permanentés | | | | cendré légèrement irisé |

## Revendications

1. Procédé de teinture des fibres kératiniques dans lequel la révélation de la couleur s'effectue uniquement au moyen de l'oxygène de l'air, caractérisé en ce que l'on applique sur les fibres :
   a) au moins une composition (A) contenant, dans un milieu approprié pour la teinture, au moins un mono- ou dihydroxyindole, cette application étant précédée ou suivie par l'application de
   b) au moins une composition (B) contenant, dans un milieu approprié pour la teinture, au moins un dérivé carbonylé aromatique choisi parmi les hydroxyacétophénones, les hydroxybenzophénones, les 2-hydroxy 1,4-benzoquinones, les hydroxy 1,4-naphtoquinones, les amino 1,4-naphtoquinones, les hydroxy 9,10-anthraquinones et les amino 9,10-anthraquinones.

2. Procédé selon la revendication 1, caractérisé en ce que les mono- ou dihydroxyindoles sont choisis parmi les composés de formule (I) :

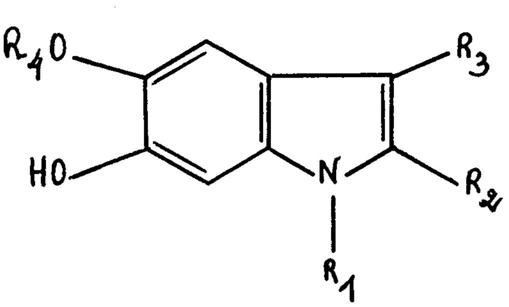

(I)

dans laquelle :

$R_1$, $R_3$ et $R_4$, identiques ou différents, représentent un atome d'hydrogène ou un groupement alkyle en $C_1$-$C_4$;

$R_2$ désigne un atome d'hydrogène, un groupement alkyle en $C_1$-$C_4$ ou un groupement carboxyle;

et

les sels correspondants de métaux alcalins, alcalino-terreux, d'ammonium et d'amines de ces composés,

le potentiel d'oxydo-réduction des composés de formule (I), mesuré à pH 7 sur électrode de carbone vitreux par voltamétrie, étant inférieur à 200 mV.

**3.** Procédé selon la revendication 1 ou 2, caractérisé en ce que les mono- ou dihydroxyindoles sont choisis parmi :

le 5,6-dihydroxyindole,
le 2-méthyl 5,6-dihydroxyindole,
le 3-méthyl 5,6-dihydroxyindole,
le 1-méthyl 5,6-dihydroxyindole,
le 2,3-diméthyl 5,6-dihydroxyindole,
le 5-méthoxy 6-hydroxyindole, et
le 2-carboxy 5,6-dihydroxyindole.

**4.** Procédé selon l'une des revendications 1 à 3, caractérisé en ce que, dans la composition (B),

les hydroxyacétophénones ou hydroxybenzophénones sont choisies parmi les composés de formule (II) :

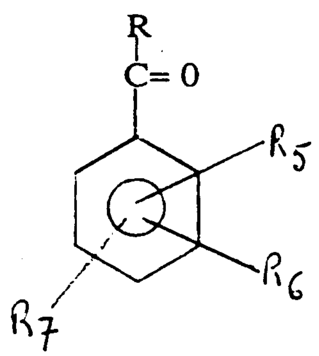

(II)

dans laquelle :

$R_5$, $R_6$, $R_7$, identiques ou différents, désignent un atome d'hydrogène, un groupement hydroxy ou méthoxy, l'un au moins parmi $R_5$, $R_6$ et $R_7$, désignant hydroxy,

R représente un groupement méthyle ou $R_8$, $R_8$ représentant un radical

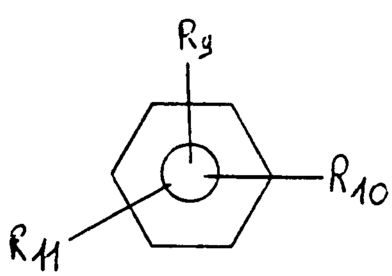

où $R_9$, $R_{10}$ et $R_{11}$, identiques ou différents, représentent un atome d'hydrogène, un groupement hydroxy ou méthoxy; et

les 2-hydroxy 1,4-benzoquinones sont choisis parmi les composés de formule (III) :

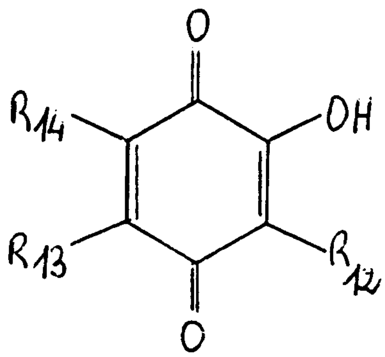

(III)

dans laquelle :

$R_{12}$, $R_{13}$ et $R_{14}$, identiques ou différents, représentent un atome d'hydrogène, un groupement alcoxy en $C_1$-$C_4$, hydroxy, alkyle en $C_1$-$C_4$, amino, aminoalkyle en $C_1$-$C_4$ ou parahydroxyphényle; et

les hydroxy 1,4-naphtoquinones ou amino 1,4-naphtoquinones sont choisies parmi les composés de formule (IV) :

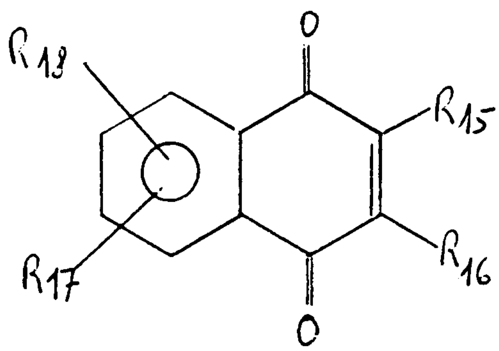

(IV)

dans laquelle :

$R_{15}$ et $R_{16}$, identiques ou différents, représentent un atome d'hydrogène ou de chlore, un groupement hydroxy, méthoxy, méthyle, isopentényle, acétyle, $-SCH_2COOH$, ou un groupement $-NR_{19}R_{20}$ , où $R_{19}$ et $R_{20}$, identiques ou différents, représentent un atome d'hydrogène ou un groupement méthyle ou hydroxyéthyle,

$R_{17}$ et $R_{18}$, identiques ou différents, représentent un atome d'hydrogène, un groupement hydroxy, méthoxy ou méthyle,

l'un au moins parmi $R_{15}$, $R_{16}$, $R_{17}$ et $R_{18}$ représentant hydroxy ou l'un au moins de $R_{15}$ et $R_{16}$ représentant $-NR_{19}R_{20}$;

les hydroxy 9,10-anthraquinones ou amino 9,10-anthraquinones sont choisies parmi les composés de formule (V) :

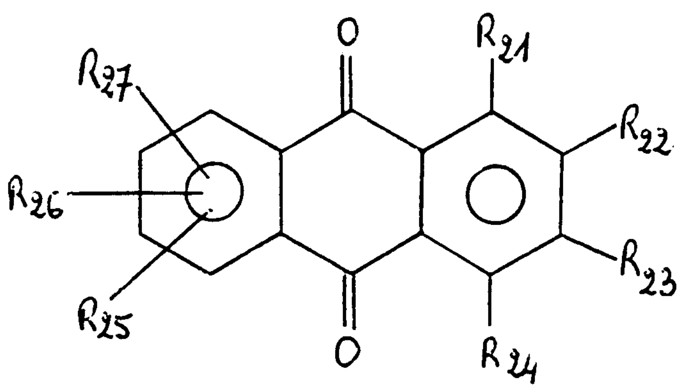

(V)

dans laquelle :

$R_{21}$ à $R_{27}$, identiques ou différents, représentent un atome d'hydrogène ou de chlore, un groupement hydroxy, méthyle, $SO_3M$, COOM ou $NR_{28}R_{29}$,

où M désigne un atome d'hydrogène, Na ou K; et

où $R_{28}$ et $R_{29}$, identiques ou différents, représentent un atome d'hydrogène, un groupement alkyle en $C_1$-$C_4$ ou monohydroxyalkyle en $C_1$-$C_4$,

l'un au moins parmi $R_{21}$ à $R_{27}$ représentant un groupement hydroxy ou $NR_{28}R_{29}$.

**5.** Procédé selon l'une des revendications 1 à 4, caractérisé en ce que la composition (B) comporte, dans un milieu approprié pour la teinture, au moins l'un des composés suivants :

la 2-hydroxyacétophénone,
la 2,5-dihydroxyacétophénone,
la 2,6-dihydroxyacétophénone,
la 4-hydroxy 3-méthoxyacétophénone,
la 3,4,5-trihydroxyacétophénone,
la 2′,2′-dihydroxybenzophénone,
la 2-hydroxybenzophénone,
la 2,2′,4,4′-tétrahydroxybenzophénone, et
la 3,4,2′,4′,6′-pentahydroxybenzophénone;
la 2,5-dihydroxy 1,4-benzoquinone,
la 2,5-dihydroxy 3-éthyl 1,4-benzoquinone,
la 2,5-dihydroxy 3,6-diméthoxy 1,4-benzoquinone,
la 2,5-dihydroxy 3-méthyl 6-isopropyl 1,4-benzoquinone,
la 2,5-dihydroxy 3,6-bis-p-hydroxyphényl 1,4-benzoquinone,
la 2-hydroxy 3-méthyl 6-méthoxy 1,4-benzoquinone,
la 2-hydroxy 3-méthyl 5,6-diméthoxy 1,4-benzoquinone,
la 2-hydroxy 3-méthoxy 6-méthyl 1,4-benzoquinone, et
la 2,3,5,6-tétrahydroxy 1,4-benzoquinone;
la 2-hydroxy 3-isopenténytl 1,4-naphtoquinone,
la 2-hydroxy 3-amino 7-méthoxy 1,4-naphtoquinone,
la 2-hydroxy 3-N-méthylamino 1,4-naphtoquinone,
la 2-hydroxy 6-méthoxy 1,4-naphtoquinone,
la 2-hydroxy 5-méthoxy 1,4-naphtoquinone,
la 2-hydroxy 8-méthoxy 1,4-naphtoquinone,
la 2-hydroxy 5,8-diméthoxy 1,4-naphtoquinone,
la 2-hydroxy 3-acétyl 1,4-naphtoquinone,
la 5-hydroxy 2-méthoxy 1,4-naphtoquinone,
la 5-hydroxy 3-diméthylamino 1,4-naphtoquinone,
la 5-hydroxy 3-méthoxy 1,4-naphtoquinone,
la 5-hydroxy 2-méthyl 1,4-naphtoquinone,
la 5-hydroxy 2-méthyl 3-chloro 1,4-naphtoquinone,
la 5-hydroxy 2-carboxyméthylthio 1,4-naphtoquinone,
la 5-hydroxy 3-carboxyméthylthio 1,4-naphtoquinone,
la 5,8-dihydroxy 1,4-naphtoquinone,
la 5,8-dihydroxy 2-chloro 1,4-naphtoquinone,
la 2,7-dihydroxy 1,4-naphtoquinone,
la 5,7-dihydroxy 1,4-naphtoquinone,
la 3,5-dihydroxy 2-méthyl 1,4-naphtoquinone,
la 3,5-dihydroxy 1,4-naphtoquinone,
la 2,6-dihydroxy 1,4-naphtoquinone,
la 2,3-dihydroxy 5-méthoxy 1,4-naphtoquinone,
la 2,3-dihydroxy 7-méthoxy 1,4-naphtoquinone,
la 3,6,8-trihydroxy 1,4-naphtoquinone,
la 2,3,5-trihydroxy 1,4-naphtoquinone,
la 2,6,7-trihydroxy 1,4-naphtoquinone,
la 2,3,5,8-tétrahydroxy 1,4-naphtoquinone, et
la 1,4-dihydroxy 9,10-anthraquinone,
la 1,5-dihydroxy 9,10-anthraquinone,

la 1,8-dihydroxy 9,10-anthraquinone,
la 2,6-dihydroxy 9,10-anthraquinone,
la 1,2,5,8-tétrahydroxy 9,10-anthraquinone,
la 1,2,3,5,6,7-hexahydroxy 9,10-anthraquinone,
la 1,8-dihydroxy 3-méthyl 9,10-anthraquinone,
la 1,3,8-trihydroxy 6-méthyl 9,10-anthraquinone,
la 5,8-dichloro 1,4-dihydroxy 9,10-anthraquinone,
le sel de sodium de l'acide 1,2-dihydroxy 9,10-anthraquinone 3-sulfonique,
le sel de sodium de l'acide 1,2,4-trihydroxy 9,10-anthraquinone 3-sulfonique,
la 1-hydroxyanthraquinone,
la 2-hydroxyanthraquinone,
la 1,8-dihydroxy 3-carboxy 9,10-anthraquinone,
la 1,4,5,8-tétraaminoanthraquinone, et
la 1,4-diaminoanthraquinone.

**6.** Procédé selon l'une des revendications 1 à 4, caractérisé en ce que la composition (B) comporte au moins l'un des composés suivants :
- la 5,8-dihydroxy 1,4-naphtoquinone,
- la 1,2,5,8-tétrahydroxyanthraquinone, et
- la 1,4,5,8-tétraaminoanthraquinone.

**7.** Procédé selon l'une des revendications 1 à 4, caractérisé en ce que la composition (B) comporte au moins l'un des composés suivants :
la 2,5-dihydroxy 3-méthyl 1,4-benzoquinone,
la 2,5-dihydroxy 3-méthoxy 6-méthyl 1,4-benzoquinone,
la 2-hydroxy 1,4-naphtoquinone,
la 2-hydroxy 3-méthyl 1,4-naphtoquinone,
la 2,3-dihydroxy 1,4-naphtoquinone,
la 2,5,7-trihydroxy 1,4-naphtoquinone,
la 5-hydroxy 1,4-naphtoquinone,
la 2,5,8-trihydroxy 1,4-naphtoquinone,
la 2-hydroxy 3-méthoxy 1,4-naphtoquinone,
la 2-carboxy 1-méthyl 3,5,6,8-tétrahydroxy anthraquinone,
la 1,2-dihydroxyanthraquinone,
la 1,2,4-trihydroxyanthraquinone,
la 3-carboxy 1,2,4-trihydroxyanthraquinone, et
la 2,5-dihydroxy 1,4-naphtoquinone.

**8.** Procédé selon l'une des revendications 1 à 7, caractérisé en ce que le mono- ou dihydroxyindole est présent dans la composition (A) en une concentration comprise entre 0,05 et 5% en poids, de préférence entre 0,05 et 3% en poids par rapport au poids total de la composition (A).

**9.** Procédé selon l'une des revendications 1 à 8, caractérisé en ce que, dans la composition (B), la concentration en composés de formules (II), (III), (IV) ou (V) est comprise entre 0,01 et 5% en poids, de préférence 0,05 et 3% en poids par rapport au poids total de la composition (B).

**10.** Procédé selon l'une des revendications 1 à 9, caractérisé en ce que les compositions (A) et (B) comprennent un milieu aqueux constitué par de l'eau ou un mélange d'eau et de solvant(s).

**11.** Procédé selon l'une des revendications 1 à 9, caractérisé en ce que les compositions (A) et/ou (B) sont constituées par un milieu solvant anhydre.

**12.** Procédé selon l'une des revendications 1 à 9, caractérisé en ce que la composition (B) est constituée par un milieu solvant anhydre, comportant au moins 15% en poids d'un solvant anhydre et au moins 20% en poids d'un ou plusieurs agents tensio-actifs anhydres.

**13.** Procédé selon l'une des revendications 1 à 9, caractérisé en ce que la composition (B) est sous forme de poudre à diluer au moment de l'emploi.

**14.** Procédé selon l'une des revendications 12 ou 13, caractérisé en ce que l'on dilue la composition (A) et/ou (B) au moment de l'emploi.

**15.** Procédé selon l'une des revendications 1 à 14, caractérisé en ce que les compositions (A) et/ou (B) contiennent, indépendamment l'une de l'autre, des agents tensio-actifs anioniques, cationiques, non-ioniques, amphotères ou leurs mélanges, des agents épaississants, des parfums, des agents séquestrants, des agents filmogènes, des agents de traitement des fibres, des agents dispersants, des agents de conditionnement, des agents conservateurs, des agents opacifiants, des agents de gonflement des fibres kératiniques, des agents de pénétration, des agents antioxydants, et des tampons.

**16.** Procédé selon l'une des revendications 1 à 13, caractérisé en ce que l'application sur les fibres kératiniques de la composition (A) est réalisée pendant 2 à 60 minutes, de préférence 5 à 30 minutes, éventuellement suivie par un rinçage, puis l'application de la composition (B) est réalisée pendant 5 à 60 minutes, de préférence 10 à 40 minutes.

**17.** Agent de teinture des fibres kératiniques, en particulier des cheveux, destiné à être utilisé dans le procédé selon l'une quelconque des revendications 1 à 15, caractérisé en ce qu'il comporte les compositions (A) et (B) telles que définies aux revendications 1 à 15, sous forme séparée, les compositions (A) et (B) étant destinées à être appliquées de façon successive sur les fibres à traiter.

**18.** Dispositif à plusieurs compartiments ou "kit de teinture" destiné à la mise en oeuvre du procédé selon l'une quelconque des revendications 1 à 15, caractérisé en ce qu'il comporte au moins deux compartiments, l'un renfermant la composition (A) telle que définie dans l'une des revendications 1 à 3, 8 et 11, et le second renferme la composition (B) telle que définie dans l'une quelconque des revendications 1 et 4 à 13, et éventuellement un troisième compartiment contenant un milieu aqueux cosmétiquement acceptable, approprié pour la teinture, destiné à être mélangé avant l'emploi à l'un et/ou l'autre des contenus des deux premiers compartiments.

**Claims**

**1.** Method for dyeing keratinous fibres, in which method the colour is developed solely by means of atmospheric oxygen, characterised in that the following are applied to the fibres:
a) a composition (A) containing, in a medium appropriate for dyeing, at least one monohydroxyindole or dihydroxyindole, this application being preceded or followed by the application of
b) a composition (B) containing, in a medium appropriate for dyeing, at least one aromatic carbonyl derivative chosen from hydroxyacetophenones, hydroxybenzophenones, 2-hydroxy-1,4-benzoquinones, hydroxy-1,4-naphthoquinones, amino-1,4-naphthoquinones, hydroxy-9,10-anthraquinones and amino-9,10-anthraquinones.

**2.** Method according to Claim 1, characterised in that the monohydroxyindoles or dihydroxyindoles are chosen from the compounds of formula (I):

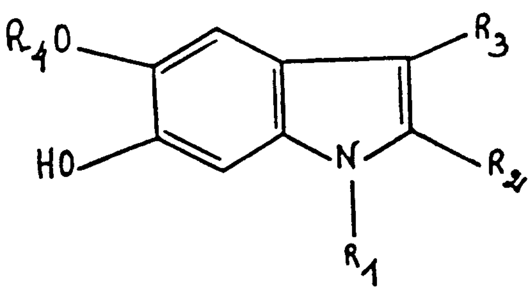

(I)

in which:
$R_1$, $R_3$ and $R_4$, which may be identical or different, represent a hydrogen atom or a $C_1$-$C_4$ alkyl group;
$R_2$ denotes a hydrogen atom, a $C_1$-$C_4$ alkyl group or a carboxyl group; and
the corresponding alkali metal, alkaline earth metal, ammonium and amine salts of these compounds,
the redox potential of the compounds of formula (I), measured at pH 7 on a vitreous carbon electrode

by voltammetry, being less than 200 mV.

3. Method according to Claim 1 or 2, characterised in that the monohydroxyindoles or dihydroxyindoles are chosen from:
5,6-dihydroxyindole,
2-methyl-5,6-dihydroxyindole,
3-methyl-5,6-dihydroxyindole,
1-methyl-5,6-dihydroxyindole,
2,3-dimethyl-5,6-dihydroxyindole,
5-methoxy-6-hydroxyindole and
2-carboxy-5,6-dihydroxyindole.

4. Method according to one of Claims 1 to 3, characterised in that, in the composition (B), the hydroxyacetophenones or hydroxybenzophenones are chosen from the compounds of formula (II):

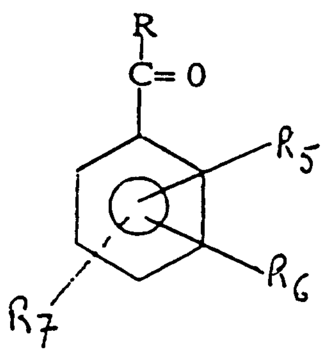

(II)

in which:
$R_5$, $R_6$ and $R_7$, which may be identical or different, denote a hydrogen atom or a hydroxyl or methoxy group, at least one of $R_5$, $R_6$ and $R_7$ denoting hydroxyl, and
R represents a methyl group or $R_8$, $R_8$ representing a

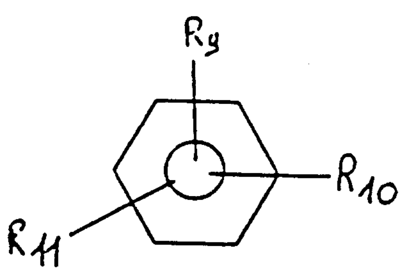

radical, where $R_9$, $R_{10}$ and $R_{11}$, which may be identical or different, represent a hydrogen atom or a hydroxyl or methoxy group; and
the 2-hydroxy-1,4-benzoquinones are chosen from the compounds of formula (III):

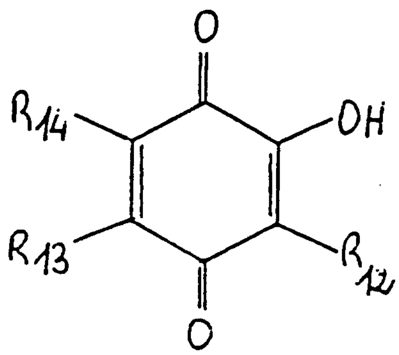

(III)

in which:

$R_{12}$, $R_{13}$ and $R_{14}$, which may be identical or different, represent a hydrogen atom or a $C_1$-$C_4$ alkoxy, hydroxyl, $C_1$-$C_4$ alkyl, amino, $C_1$-$C_4$ aminoalkyl or parahydroxyphenyl group; and

the hydroxy-1,4-naphthoquinones or amino-1,4-naphthoquinones are chosen from the compounds of formula (IV):

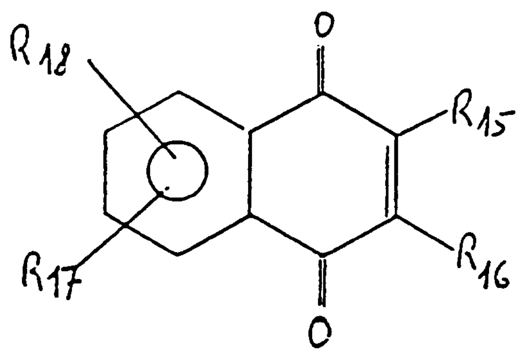

(IV)

in which:

$R_{15}$ and $R_{16}$, which may be identical or different, represent a hydrogen or chlorine atom or a hydroxyl, methoxy, methyl, isopentenyl, acetyl or $-SCH_2COOH$ group, or a $-NR_{19}R_{20}$ group, where $R_{19}$ and $R_{20}$, which may be identical or different, represent a hydrogen atom or a methyl or hydroxyethyl group, and

$R_{17}$ and $R_{18}$, which may be identical or different, represent a hydrogen atom or a hydroxyl, methoxy or methyl group, at least one of $R_{15}$, $R_{16}$, $R_{17}$ and $R_{18}$ representing hydroxyl or at least one of $R_{15}$ and $R_{16}$ representing $-NR_{19}R_{20}$; and the hydroxy-9,10-anthraquinones or amino-9,10-anthraquinones are chosen from the compounds of formula (V):

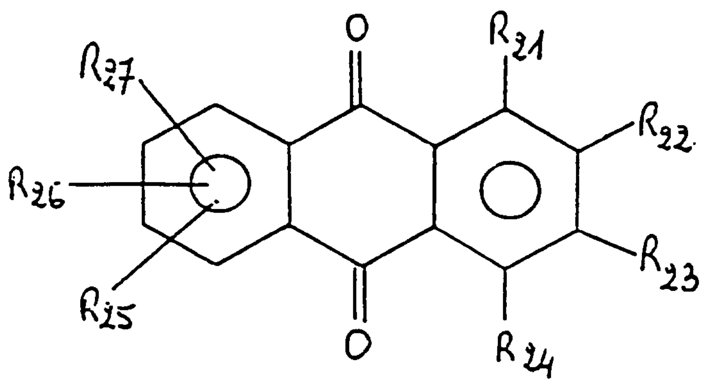

(V)

in which:

$R_{21}$ to $R_{27}$, which may be identical or different, represent a hydrogen or chlorine atom or a hydroxyl, methyl, $SO_3M$, COOM or $NR_{28}R_{29}$ group,

where M denotes a hydrogen, Na or K atom, and
where $R_{28}$ and $R_{29}$, which may be identical or different, represent a hydrogen atom or a $C_1$-$C_4$ alkyl or $C_1$-$C_4$ mono-hydroxyalkyl group,
at least one of $R_{21}$ to $R_{27}$ representing a hydroxyl or $NR_{28}R_{29}$ group.

**5.** Method according to one of Claims 1 to 4, characterised in that the composition (B) comprises, in a medium appropriate for dyeing, at least one of the following compounds:
2-hydroxyacetophenone,
2,5-dihydroxyacetophenone,
2,6-dihydroxyacetophenone,
4-hydroxy-3-methoxyacetophenone,
3,4,5-trihydroxyacetophenone,
2',2'-dihydroxybenzophenone,
2-hydroxybenzophenone,
2,2',4,4'-tetrahydroxybenzophenone and
3,4,2',4',6'-pentahydroxybenzophenone;
2,5-dihydroxy-1,4-benzoquinone,
2,5-dihydroxy-3-ethyl-1,4-benzoquinone,
2,5-dihydroxy-3,6-dimethoxy-1,4-benzoquinone,
2,5-dihydroxy-3-methyl-6-isopropyl-1,4-benzoquinone,
2,5-dihydroxy-3,6-bis-p-hydroxyphenyl-1,4-benzoquinone,
2-hydroxy-3-methyl-6-methoxy-1,4-benzoquinone,
2-hydroxy-3-methyl-5,6-dimethoxy-1,4-benzoquinone,
2-hydroxy-3-methoxy-6-methyl-1,4-benzoquinone and
2,3,5,6-tetrahydroxy-1,4-benzoquinone;
2-hydroxy-3-isopentenyl-1,4-naphthoquinone,
2-hydroxy-3-amino-7-methoxy-1,4-naphthoquinone,
2-hydroxy-3-N-methylamino-1,4-naphthoquinone,
2-hydroxy-6-methoxy-1,4-naphthoquinone,
2-hydroxy-5-methoxy-1,4-naphthoquinone,
2-hydroxy-8-methoxy-1,4-naphthoquinone,
2-hydroxy-5,8-dimethoxy-1,4-naphthoquinone,
2-hydroxy-3-acetyl-1,4-naphthoquinone,
5-hydroxy-2-methoxy-1,4-naphthoquinone,
5-hydroxy-3-dimethylamino-1,4-naphthoquinone,
5-hydroxy-3-methoxy-1,4-naphthoquinone,
5-hydroxy-2-methyl-1,4-naphthoquinone,
5-hydroxy-2-methyl-3-chloro-1,4-naphthoquinone,
5-hydroxy-2-carboxymethylthio-1,4-naphthoquinone,
5-hydroxy-3-carboxymethylthio-1,4-naphthoquinone,
5,8-dihydroxy-1,4-naphthoquinone,
5,8-dihydroxy-2-chloro-1,4-naphthoquinone,
2,7-dihydroxy-1,4-naphthoquinone,
5,7-dihydroxy-1,4-naphthoquinone,
3,5-dihydroxy-2-methyl-1,4-naphthoquinone,
3,5-dihydroxy-1,4-naphthoquinone,
2,6-dihydroxy-1,4-naphthoquinone,
2,3-dihydroxy-5-methoxy-1,4-naphthoquinone,
2,3-dihydroxy-7-methoxy-1,4-naphthoquinone,
3,6,8-trihydroxy-1,4-naphthoquinone,
2,3,5-trihydroxy-1,4-naphthoquinone,
2,6,7-trihydroxy-1,4-naphthoquinone,
2,3,5,8-tetrahydroxy-1,4-naphthoquinone and
1,4-dihydroxy-9,10-anthraquinone,
1,5-dihydroxy-9,10-anthraquinone,
1,8-dihydroxy-9,10-anthraquinone,
2,6-dihydroxy-9,10-anthraquinone,
1,2,5,8-tetrahydroxy-9,10-anthraquinone,

1,2,3,5,6,7-hexahydroxy-9,10-anthraquinone,
1,8-dihydroxy-3-methyl-9,10-anthraquinone,
1,3,8-trihydroxy-6-methyl-9,10-anthraquinone,
5,8-dichloro-1,4-dihydroxy-9,10-anthraquinone,
the sodium salt of 1,2-dihydroxy-9,10-anthraquinone-3-sulphonic acid,
the sodium salt of 1,2,4-trihydroxy-9,10-anthraquinone-3-sulphonic acid,
1-hydroxyanthraquinone,
2-hydroxyanthraquinone,
1,8-dihydroxy-3-carboxy-9,10-anthraquinone,
1,4,5,8-tetraaminoanthraquinone and
1,4-diaminoanthraquinone.

**6.** Method according to one of Claims 1 to 4, characterised in that the composition (B) comprises at least one of the following compounds:
- 5,8-dihydroxy-1,4-naphthoquinone,
- 1,2,5,8-tetrahydroxyanthraquinone and
- 1,4,5,8-tetraaminoanthraquinone.

**7.** Method according to one of Claims 1 to 4, characterised in that the composition (B) comprises at least one of the following compounds:
2,5-dihydroxy-3-methyl-1,4-benzoquinone,
2,5-dihydroxy-3-methoxy-6-methyl-1,4-benzoquinone,
2-hydroxy-1,4-naphthoquinone,
2-hydroxy-3-methyl-1,4-naphthoquinone,
2,3-dihydroxy-1,4-naphthoquinone,
2,5,7-trihydroxy-1,4-naphthoquinone,
5-hydroxy-1,4-naphthoquinone,
2,5,8-trihydroxy-1,4-naphthoquinone,
2-hydroxy-3-methoxy-1,4-naphthoquinone,
2-carboxy-1-methyl-3,5,6,8-tetrahydroxy-anthraquinone,
1,2-dihydroxyanthraquinone,
1,2,4-trihydroxyanthraquinone,
3-carboxy-1,2,4-trihydroxyanthraquinone and
2,5-dihydroxy-1,4-naphthoquinone.

**8.** Method according to one of Claims 1 to 7, characterised in that the monohydroxyindole or dihydroxyindole is present in the composition (A) in a concentration of between 0.05 and 5% by weight and preferably between 0.05 and 3% by weight with respect to the total weight of the composition (A).

**9.** Method according to one of Claims 1 to 8, characterised in that, in the composition (B), the concentration of compounds of formulae (II), (III), (IV) or (V) is between 0.01 and 5% by weight and preferably 0.05 and 3% by weight with respect to the total weight of the composition (B).

**10.** Method according to one of Claims 1 to 9, characterised in that the compositions (A) and (B) contain an aqueous medium consisting of water or a mixture of water and solvent(s).

**11.** Method according to one of Claims 1 to 9, characterised in that the compositions (A) and/or (B) consist of an anhydrous solvent medium.

**12.** Method according to one of Claims 1 to 9, characterised in that the composition (B) consists of an anhydrous solvent medium containing at least 15% by weight of an anhydrous solvent and at least 20% by weight of one or more anhydrous surfactants.

**13.** Method according to one of Claims 1 to 9, characterised in that the composition (B) is in the form of a powder to be diluted at the time of use.

**14.** Method according to either of Claims 12 or 13, characterised in that the composition (A) and/or (B) is diluted at the time of use.

**15.** Method according to one of Claims 1 to 14, characterised in that the compositions (A) and/or (B) contain, independently of one another, anionic, cationic, nonionic or amphoteric surfactants or mixtures thereof, thickeners, perfumes, sequestering agents, film-forming agents, fibre-treatment agents, dispersing agents, conditioners, preservatives, opacifying agents, agents for swelling keratinous fibres, penetrating agents, antioxidants and buffers.

**16.** Method according to one of Claims 1 to 13, characterised in that the composition (A) is applied to the keratinous fibres for 2 to 60 minutes, preferably 5 to 30 minutes, this application optionally being followed by rinsing, and the composition (B) is then applied for 5 to 60 minutes, preferably 10 to 40 minutes.

**17.** Dyeing agent for keratinous fibres, in particular the hair, which is intended to be used in the method according to any one of Claims 1 to 15, characterised in that it comprises the compositions (A) and (B) as defined in Claims 1 to 15, as separate components, the compositions (A) and (B) being intended to be applied successively to the fibres to be treated.

**18.** Multi-compartment device or "dyeing kit" intended for carrying out the method according to any one of Claims 1 to 15, characterised in that it comprises at least two compartments, one containing the composition (A) as defined in one of Claims 1 to 3, 8 and 11, and the second contains the composition (B) as defined in any one of Claims 1 and 4 to 13, and optionally a third compartment contains a cosmetically acceptable aqueous medium appropriate for dyeing and intended for mixing, before use, with the contents of one and/or the other of the first two compartments.

**Patentansprüche**

**1.** Verfahren zur Färbung keratinischer Fasern, wobei die Entwicklung der Farbe alleiniglich mittels des Sauerstoffs der Luft erfolgt,
dadurch **gekennzeichnet**, daß
man auf die Fasern:
a) mindestens eine Zusammensetzung (A) aufbringt, die, in einem zur Färbung geeigneten Milieu, mindestens ein Mono- oder Dihydroxyindol enthält, wobei vor oder nach dieser Aufbringung die Aufbringung
b) mindestens einer Zusammensetzung (B) erfolgt, die, in einem zur Färbung geeigneten Milieu, mindestens ein aromatisches Carbonylderivat enthält, das aus Hydroxyacetophenonen, Hydroxybenzophenonen, 2-Hydroxy-1,4-benzochinonen, Hydroxy-1,4-naphthochinonen, Amino-1,4-naphthochinonen, Hydroxy-9,10-anthrachinonen und Amino-9,10-anthrachinonen ausgewählt ist.

**2.** Verfahren gemäß Anspruch 1,
dadurch **gekennzeichnet**, daß
die Mono- oder Dihydroxyindole aus den Verbindungen der Formel (I):

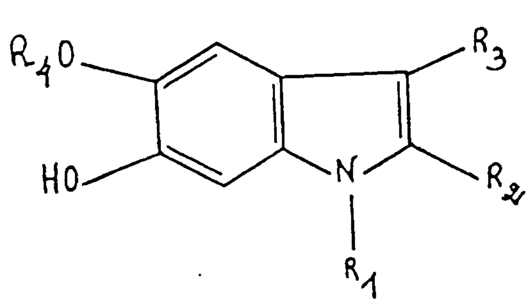

(I)

worin gilt:
$R_1$, $R_3$ und $R_4$ stellen, gleich oder verschieden, ein Wasserstoffatom oder eine $C_{1-4}$-Alkylgruppe dar;
$R_2$ bedeutet ein Wasserstoffatom, eine $C_{1-4}$-Alkyl- oder Carboxylgruppe;
sowie aus den entsprechenden Alkali-, Erdalkali-, Ammonium- und Aminsalzen dieser Verbindungen ausgewählt sind, wobei das Redoxpotential der Verbindungen der Formel (I), gemessen bei pH 7 durch Voltametrie an einer Elektrode von glasartigem Kohlenstoff, weniger als 200 mV beträgt.

**3.** Verfahren gemäß Anspruch 1 oder 2,
dadurch **gekennzeichnet**, daß
die Mono- oder Dihydroxyindole ausgewählt sind aus:
5,6-Dihydroxyindol,
2-Methyl-5,6-dihydroxyindol,
3-Methyl-5,6-dihydroxyindol,
1-Methyl-5,6-dihydroxyindol,
2,3-Dimethyl-5,6-dihydroxyindol,
5-Methoxy-6-hydroxyindol und
2-Carboxy-5,6-dihydroxyindol.

**4.** Verfahren gemäß einem der Ansprüche 1 bis 3,
dadurch **gekennzeichnet**, daß,
in der Zusammensetzung (B), die Hydroxyacetophenone oder Hydroxybenzophenone aus den Verbindungen der Formel (II) ausgewählt sind:

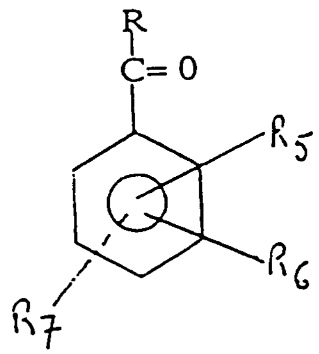

(II)

worin gilt:
$R_5$, $R_6$ und $R_7$ bedeuten, gleich oder verschieden, ein Wasserstoffatom, eine Hydroxy- oder Methoxygruppe, wobei mindestens einer der Reste $R_5$, $R_6$ und $R_7$ einen Hydroxyrest darstellt,
R stellt eine Methylgruppe oder den Rest $R_8$ dar, wobei $R_8$ einen Rest darstellt:

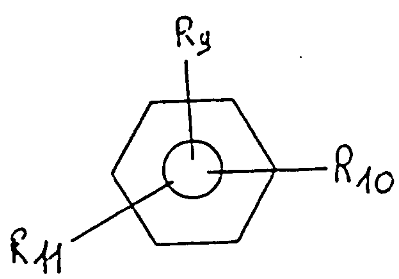

worin $R_9$, $R_{10}$ und $R_{11}$, gleich oder verschieden, ein Wasserstoffatom, eine Hydroxy- oder Methoxygruppe darstellen,
die 2-Hydroxy-1,4-benzochinonen aus den Verbindungen der Formel (III) ausgewählt sind:

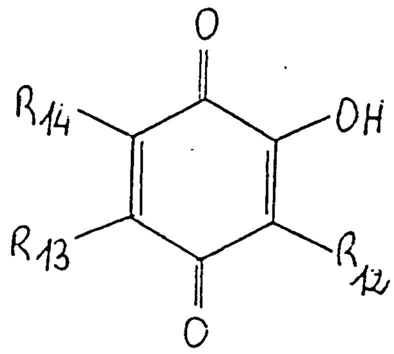

(III)

worin gilt:
$R_{12}$, $R_{13}$ und $R_{14}$ stellen, gleich oder verschieden, ein Wasserstoffatom, eine $C_{1-4}$-Alkoxy-, $C_{1-4}$-Alkyl-, Amino-, Amino-$C_{1-4}$-alkyl- oder p-Hydroxyphenylgruppe dar,
die Hydroxy-1,4-naphthochinone oder Amino-1,4-naphthochinone aus den Verbindungen der Formel (IV) ausgewählt sind:

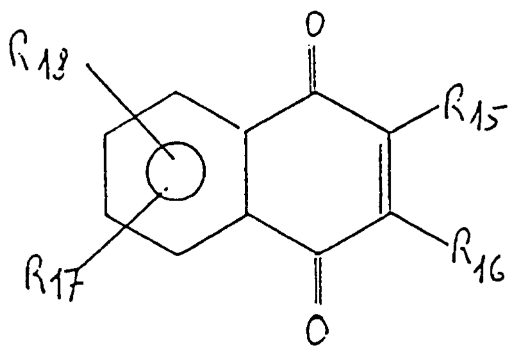

(IV)

worin gilt:
$R_{15}$ und $R_{16}$ stellen, gleich oder verschieden, ein Wasserstoff- oder Chloratom, eine Hydroxy-, Methoxy-, Methyl-, Isopentenyl-, Acetyl-, $-SCH_2COOH$- oder eine $-NR_{19}R_{20}$-Gruppe dar, worin $R_{19}$ und $R_{20}$, gleich oder verschieden, ein Wasserstoffatom oder eine Methyl- oder Hydroxyethylgruppe darstellen,
$R_{17}$ und $R_{18}$ stellen, gleich oder verschieden, ein Wasserstoffatom, eine Hydroxy-, Methoxy- oder Methylgruppe dar,
wobei mindestens einer der Reste $R_{15}$, $R_{16}$, $R_{17}$ und $R_{18}$ eine Hydroxygruppe oder mindestens einer der Reste $R_{15}$ und $R_{16}$ eine $-NR_{19}R_{20}$-Gruppe darstellen, und daß
die Hydroxy-9,10-anthrachinone oder Amino-9,10-anthrachinone aus den Verbindungen der Formel (V) ausgewählt sind:

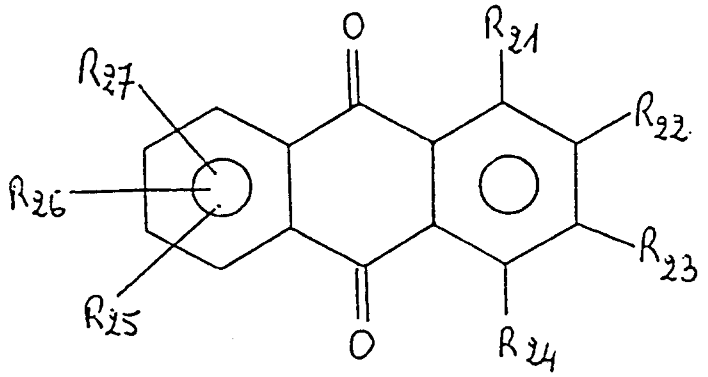

(V)

worin gilt:
$R_{21}$ bis $R_{27}$ stellen, gleich oder verschieden, ein Wasserstoff- oder Chloratom, eine Hydroxy-, Methyl-, $-SO_3M$-, -COOM- oder $-NR_{28}R_{29}$-Gruppe dar,
worin M ein Wasserstoffatom, Na oder K bedeutet, und worin $R_{28}$ und $R_{29}$, gleich oder verschieden, ein Wasserstoffatom, eine $C_{1-4}$-Alkyl- oder Monohydroxy-$C_{1-4}$-alkylgruppe darstellen,
wobei mindestens einer der Reste $R_{21}$ bis $R_{27}$ eine Hydroxy- oder $-NR_{28}R_{29}$-Gruppe darstellt.

**5.** Verfahren gemäß einem der Ansprüche 1 bis 4,
dadurch **gekennzeichnet**, daß
die Zusammensetzung (B), in einem zur Färbung geeigneten Milieu, mindestens eine der folgenden Verbindungen enthält:
2-Hydroxyacetophenon,
2,5-Dihydroxyacetophenon,
2,6-Dihydroxyacetophenon,
4-Hydroxy-3-methoxyacetophenon,
3,4,5-Trihydroxyacetophenon,
2,2'-Dihydroxybenzophenon,
2-Hydroxybenzophenon,
2,2',4,4'-Tetrahydroxybenzophenon und
3,4,2',4',6'-Pentahydroxybenzophenon;
2,5-Dihydroxy-1,4-benzochinon,
2,5-Dihydroxy-3-ethyl-1,4-benzochinon,
2,5-Dihydroxy-3,6-dimethoxy-1,4-benzochinon,
2,5-Dihydroxy-3-methyl-6-isopropyl-1,4-benzochinon,
2,5-Dihydroxy-3,6-bis-p-hydroxyphenyl-1,4-benzochinon,
2-Hydroxy-3-methyl-6-methoxy-1,4-benzochinon,
2-Hydroxy-3-methyl-5,6-dimethoxy-1,4-benzochinon,
2-Hydroxy-3-methoxy-6-methyl-1,4-benzochinon und
2,3,5,6-Tetrahydroxy-1,4-benzochinon;
2-Hydroxy-3-isopentenyl-1,4-naphthochinon,
2-Hydroxy-3-amino-7-methoxy-1,4-naphthochinon,
2-Hydroxy-3-N-methylamino-1,4-naphthochinon,
2-Hydroxy-6-methoxy-1,4-naphthochinon,
2-Hydroxy-5-methoxy-1,4-naphthochinon,
2-Hydroxy-8-methoxy-1,4-naphthochinon,
2-Hydroxy-5,8-dimethoxy-1,4-naphthochinon,
2-Hydroxy-3-acetyl-1,4-naphthochinon,
5-Hydroxy-2-methoxy-1,4-naphthochinon,
5-Hydroxy-3-dimethylamino-1,4-naphthochinon,
5-Hydroxy-3-methoxy-1,4-naphthochinon,
5-Hydroxy-2-methyl-1,4-naphthochinon,
5-Hydroxy-2-methyl-3-chlor-1,4-naphthochinon,
5-Hydroxy-2-carboxymethylthio-1,4-naphthochinon,
5-Hydroxy-3-carboxymethylthio-1,4-naphthochinon,
5,8-Dihydroxy-1,4-naphthochinon,
5,8-Dihydroxy-2-chlor-1,4-naphthochinon,
2,7-Dihydroxy-1,4-naphthochinon,
5,7-Dihydroxy-1,4-naphthochinon,
3,5-Dihydroxy-2-methyl-1,4-naphthochinon,
3,5-Dihydroxy-1,4-naphthochinon,
2,6-Dihydroxy-1,4-naphthochinon,
2,3-Dihydroxy-5-methoxy-1,4-naphthochinon,
2,3-Dihydroxy-7-methoxy-1,4-naphthochinon,
3,6,8-Trihydroxy-1,4-naphthochinon,
2,3,5-Trihydroxy-1,4-naphthochinon,
2,6,7-Trihydroxy-1,4-naphthochinon,
2,3,5,8-Tetrahydroxy-1,4-naphthochinon und
1,4-Dihydroxy-9,10-anthrachinon,

1,5-Dihydroxy-9,10-anthrachinon,
1,8-Dihydroxy-9,10-anthrachinon,
2,6-Dihydroxy-9,10-anthrachinon,
1,2,5,8-Tetrahydroxy-9,10-anthrachinon,
1,2,3,5,6,7-Hexahydroxy-9,10-anthrachinon,
1,8-Dihydroxy-3-methyl-9,10-anthrachinon,
1,3,8-Trihydroxy-6-methyl-9,10-anthrachinon,
5,8-Dichlor-1,4-dihydroxy-9,10-anthrachinon,
Natrium-1,2-dihydroxy-9,10-anthrachinon-3-sulfonat,
Natrium-1,2,4-trihydroxy-9,10-anthrachinon-3-sulfonat,
1-Hydroxyanthrachinon,
2-Hydroxyanthrachinon,
1,8-Dihydroxy-3-carboxy-9,10-anthrachinon,
1,4,5,8-Tetraaminoanthrachinon und
1,4-Diaminoanthrachinon.

**6.** Verfahren gemäß einem der Ansprüche 1 bis 4,
dadurch **gekennzeichnet**, daß
die Zusammensetzung (B) mindestens eine der folgenden Verbindungen enthält:
5,8-Dihydroxy-1,4-naphthochinon,
1,2,5,8-Tetrahydroxyanthrachinon und
1,4,5,8-Tetraaminoanthrachinon.

**7.** Verfahren gemäß einem der Ansprüche 1 bis 4,
dadurch **gekennzeichnet**, daß
die Zusammensetzung (B) mindestens eine der folgenden Verbindungen enthält:
2,5-Dihydroxy-3-methyl-1,4-benzochinon,
2,5-Dihydroxy-3-methoxy-6-methyl-1,4-benzochinon,
2-Hydroxy-1,4-naphthochinon,
2-Hydroxy-3-methyl-1,4-naphthochinon,
2,5-Dihydroxy-1,4-naphthochinon,
2,3-Dihydroxy-1,4-naphthochinon,
2,5,7-Trihydroxy-1,4-naphthochinon,5-Hydroxy-1,4-naphthochinon,
2,5,8-Trihydroxy-1,4-naphthochinon,
2-Hydroxy-3-methoxy-1,4-naphthochinon
2-Carboxy-1-methyl-3,5,6,8-tetrahydroxyanthrachinon,
1,2-Dihydroxyanthrachinon,
1,2,4-Trihydroxyanthrachinon und
3-Carboxy-1,2,4-trihydroxyanthrachinon.

**8.** Verfahren gemäß einem der Ansprüche 1 bis 7,
dadurch **gekennzeichnet**, daß
das Mono- oder Dihydroxyindol in der Zusammensetzung (A) in einer Konzentration von 0,05 bis 5 und vorzugsweise von 0,05 bis 3 Gew.% vorhanden ist, bezogen auf das Gesamtgewicht der Zusammensetzung (A).

**9.** Verfahren gemäß einem der Ansprüche 1 bis 8,
dadurch **gekennzeichnet**, daß,
in der Zusammensetzung (B), die Konzentration an Verbindungen der Formeln (II), (III), (IV) oder (V) 0,01 bis 5 und vorzugsweise 0,05 bis 3 Gew.% beträgt, bezogen auf das Gesamtgewicht der Zusammensetzung (B).

**10.** Verfahren gemaß einem der Ansprüche 1 bis 9,
dadurch **gekennzeichnet**, daß
die Zusammensetzungen (A) und (B) ein wässriges Milieu aus Wasser oder einer Mischung aus Wasser und Lösungsmittel(n) enthalten.

**11.** Verfahren gemäß einem der Ansprüche 1 bis 9,
dadurch **gekennzeichnet**, daß
die Zusammensetzungen (A) und/oder (B) aus einem wasserfreien Lösungsmittelmilieu zusammengesetzt sind.

**12.** Verfahren gemäß einem der Ansprüche 1 bis 9,
dadurch **gekennzeichnet**, daß
die Zusammensetzung (B) aus einem wasserfreien Lösungsmittelmilieu zusammengesetzt ist, das mindestens 15 Gew.% eines wasserfreien Lösungsmittels und mindestens 20 Gew.% eines oder mehrerer wasserfreier oberflächenaktiver Mittel aufweist.

**13.** Verfahren gemäß einem der Ansprüche 1 bis 9,
dadurch **gekennzeichnet**, daß
die Zusammensetzung (B) in Form eines Pulvers zur zum Zeitpunkt der Anwendung durchzuführenden Verdünnung vorliegt.

**14.** Verfahren gemäß einem der Ansprüche 12 oder 13,
dadurch **gekennzeichnet**, daß
man die Zusammensetzung (A) und/oder (B) zum Zeitpunkt der Anwendung verdünnt.

**15.** Verfahren gemäß einem der Ansprüche 1 bis 14,
dadurch **gekennzeichnet**, daß
die Zusammensetzungen (A) und/oder (B), unabhängig voneinander, anionische, kationische, nichtionische oder amphotere oberflächenaktive Mittel oder deren Mischungen, Verdickungsmittel, Parfüm-Produkte, Sequestriermittel, filmbildende Mittel, Mittel zur Behandlung von Fasern, Dispergiermittel, Konditioniermittel, Konservierungsmittel, opak machende Mittel, Mittel zur Auflockerung keratinischer Fasern, Eindringmittel, Antioxidantien und Puffermittel enthalten.

**16.** Verfahren gemäß einem der Ansprüche 1 bis 13,
dadurch **gekennzeichnet**, daß
die Aufbringung der Zusammensetzung (A) auf die keratinischen Fasern 2 bis 60, vorzugsweise 5 bis 30, Minuten lang durchgeführt wird, worauf gegebenenfalls eine Spülung erfolgt, und dann die Aufbringung der Zusammensetzung (B) 5 bis 60, vorzugsweise 10 bis 40, Minuten lang durchgeführt wird.

**17.** Mittel zur Färbung keratinischer Fasern, insbesondere von Haaren, zur Verwendung im Verfahren gemäß einem jeden der Ansprüche 1 bis 15,
dadurch **gekennzeichnet**, daß
es die in den Ansprüchen 1 bis 15 definierten Zusammensetzungen (A) und (B), in getrennter Form, umfaßt, wobei die Zusammensetzungen (A) und (B) in aufeinanderfolgender Weise auf die zu behandelnden Fasern aufgebracht werden sollen.

**18.** Vorrichtung aus mehreren Teilstücken oder "Kit zur Färbung" zur Durchführung des Verfahrens gemäß jedem der Ansprüche 1 bis 15,
dadurch **gekennzeichnet**, daß
sie mindestens 2 Teilstücke umfaßt, wobei das eine die in einem der Ansprüche 1 bis 3, 8 und 11 definierte Zusammensetzung (A) und das zweite die in einem jeden der Ansprüche 1 und 4 bis 13 definierte Zusammensetzung (B) enthalten, und daß gegebenenfalls ein drittes Teilstück umfaßt ist, das ein kosmetisch verträgliches, zur Färbung geeignetes wässriges Milieu aufweist, um vor der Anwendung mit dem einen und/oder dem anderen der Inhaltsmengen der beiden ersteren Teilstücke vermischt zu werden.